# EUROPÄISCHE PATENTSCHRIFT

(11) **EP 2 391 674 B1**
(45) Veröffentlichungstag und Bekanntmachung des Hinweises auf die Patenterteilung: **06.01.2016**
(21) Anmeldenummer: 10702828.4
(22) Anmeldetag: 29.01.2010
(51) Int. Cl.: C08K 3/00, C08K 3/08, A61F 13/00

(54) **VERWENDUNG VON FEIN VERTEILTEN METALLPARTIKELN IN EINEM MATERIAL, EINER HAUTAUFLAGE UND EINEM ORTHOPÄDISCHEN ARTIKEL**
USE OF FINELY DISPERSED METAL PARTICLES IN A MATERIAL, A SKIN PATCH AND AN ORTHOPEDIC ARTICLE
UTILISATION DE PARTICULES MÉTALLIQUES FINEMENT DISPERSÉES DANS UN MATÉRIAU, UN PANSEMENT OU UN ARTICLE ORTHOPÉDIQUE

(30) Priorität: 30.01.2009 DE 102009006941
(43) Veröffentlichungstag der Anmeldung: 07.12.2011
(73) Patentinhaber: Otto Bock HealthCare GmbH, 37115 Duderstadt (DE)
(72) Erfinder: ANHALT, Klaus-Peter, 37434 Rhumspringe (DE)
(74) Vertreter: Lins, Edgar
(86) Internationale Anmeldenummer: PCT/EP2010/000540
(87) Internationale Veröffentlichungsnummer: WO 2010/086174

(56) Entgegenhaltungen:
- WO-A2-2005/023206
- DE-T2- 69 216 528
- DATABASE WPI Week 198946 Thomson Scientific, London, GB; AN 1989-336101 XP002582039 & JP 01 250411 A (KURARAY CO LTD) 5. Oktober 1989 (1989-10-05)
- DATABASE WPI Week 200921 Thomson Scientific, London, GB; AN 2009-B47542 XP002582263 & KR 2008 066 316 A (SKC CO LTD) 16. Juli 2008 (2008-07-16)

## Beschreibung

Die Erfindung betrifft die Kaschierung von Materialverfärbungen bei Gebrauch an der Luft oder auf der Haut, insbesondere hervorgerufen durch Kontakt mit Schweiß, speziell in einem Material, das selbst kein Metall ist und elektrisch nicht wesentlich leiten sollte und das für die Verwendung im direkten Hautkontakt mit einem Benutzer vorgesehen ist. Die Erfindung umfasst auch eine Anwendung in Polstern sowie orthopädischen Artikeln.

Es ist bekannt, dass Kunststoffprodukte durch UV-Strahlung, aber auch durch andere Umwelteinflüsse, wie durch Kontakt mit Körperflüssigkeiten und Schweiß, bei Gebrauch auf der Haut verfärben. Besonders häufig tritt bei weiß oder hell eingefärbten Kunststoffprodukten eine Vergilbung auf.

Vergilbungen und Verfärbungen treten z. B. bei orthopädischen Polstern vor allem dann auf, wenn diese hell oder hautfarben eingefärbt sind. Die Verfärbungen können dem Kunststoffteil einen verschmutzten und ästhetisch unangenehmen Eindruck geben, so dass solche Verfärbungen vermieden oder kaschiert werden müssen.

Häufig wird versucht, der Verfärbung mit einem höheren Pigmentgehalt zu begegnen. Dies bewirkt jedoch in der Regel eine Verschlechterung der mechanischen Eigenschaften des Kunststoffteils, auf die es bei medizinischen oder orthopädischen Polstern stark ankommen kann. Es verbietet sich daher, den Anteil an Pigmenten oder Farbstoffen als Zuschlagsstoffe so drastisch zu erhöhen, dass Verfärbungen dadurch vollständig vermieden werden könnten.

Bei Standardeinfärbungen wird das eingefärbte Kunststoffteil durch Absorption des Lichtes aus beiden Effekten, dem gewollten Effekt des Farbpigments und der ungewollten Verfärbung, im Laufe der Zeit dunkler. Eine Kaschierung der eigentlichen Verfärbung wird außerdem nur unter Verwendung einer geeigneten Farbe erreicht, welche ein Absorptionsspektrum aufweist, wo auch die Verfärbungsfarbe absorbiert.

Es besteht daher ein Bedarf an alternativ eingefärbten Kunststoffteilen für die Medizin- und Orthopädietechnik. Sowohl für medizinische wie auch spezielle orthopädische Zwecke werden zahlreiche Kunststoffteile benötigt, die im direkten Körperkontakt zum Benutzer stehen. Hierzu zählen verschiedene Polster, Orthesenteile, Prothesenteile und anderes orthopädisches Zubehör. Die DE 692 16 528 T2 beschreibt thermoplastische Harzzusammensetzungen, bei denen unterschiedliche Metallpartikel verwendet werden, um die Verfärbungen des Harzes durch die Einwirkung von Licht zu vermeiden. Das Harz wird beispielsweise für Automobilteile, wie beispielsweise Türbleche, Seitenverkleidungen oder Kofferraumdeckel, verwendet.

Die WO 2005/023206 A2 offenbart ein Körperpflegemittel, das Silber und Zink enthält, um bei Kontakt mit einer Körperflüssigkeit Zinkionen und Silberionen freizusetzen. Dabei sollen sowohl die Zink- als auch die Silberionen antibakteriell wirken.

Die JP 1 250 411 A beschreibt den Zusatz von Silber, Zink, Blei oder Eisen in Form von Pulver zu einem Polysiloxane. Die KR 2008 006 6316 beschreibt ein Verpackungsmaterial aus weichem Polyurethanschaum, der Nanopartikel aus Edelmetallen enthält.

Weiterhin ist es bekannt, Materialien mit Silber auszurüsten, um ihnen einen antibakteriellen Effekt zu verleihen, insbesondere wenn das Material für einen längeren und intensiven Hautkontakt vorgesehen ist. Die Silberausrüstungen werden besonders gern für Materialien, die mit Schweiß in Verbindung kommen, verwendet, um Geruchsbildung durch bakterielle Zersetzungsprodukte im Schweiß zu verhindern, oder auch im medizinischen Bereich für Wundauflagen und bei der Behandlung bestimmter Hauterkrankungen, wie Neurodermitis.

Hierfür ist es beispielsweise bekannt, Textilien mit Silberfäden auszurüsten oder mit einer silberhaltigen Beschichtung zu versehen. Oberflächenbeschichtungen können auch vorgesehen sein für Kühlschränke, Küchenmöbel und technische Teile, die häufig von verschiedenen Personen berührt werden, wie Lichtschalter oder Türklinken - besonders in Krankenhäusern. Bei den Beschichtungen kann es sich um dünne Öl- oder Polymerfilme handeln. Auch antibakterielle Emaillierungen und Keramiken sind bekannt, ebenso wie silberhaltige Kunststoffe zur Anwendung in der Medizin- und Orthopädietechnik.

Die antibakterielle Wirkung des Silbers wird den Silberionen zugeschrieben, die gerade im Hautkontakt durch Hautflüssigkeiten und Transpiration leicht entstehen. Im Verlaufe des Gebrauchs oxidiert das Silber teilweise. Bei Verwendung auf der Haut entsteht schwarzes Silbersulfid, das das mit Silber ausgerüstete Produkt häufig unansehnlich werden lässt. Für mit Silber ausgerüstete Textilien und Polymere werden daher die Farben Grau, Anthrazit und Schwarz bevorzugt, in denen sich bildendes Silberoxid oder -sulfid nicht auffällt. Probleme treten jedoch bei hellen oder hautfarben eingefärbten Gummi- oder Kunststoffpolstern auf, z.B. im Orthopädiebereich an Prothesen und anderen orthopädischen Hilfsmitteln.

Als Anschlussmittel und Polster zwischen Gliedmaßen-Stümpfen und zugehörigen Prothesenschäften sind z. B. sogenannte Liner im Gebrauch, die aus weichen elastischen Polymeren (Kunststoffe-, Homo- oder Copolymere, Polymergemische, Gummiarten) hergestellt werden. Unter anderem sind Silikonliner und Polyurethanliner im Handel. Dabei sind die Silikon- und Polyurethanmaterialien einschließlich von Silikon- und Polyurethanpolymergelen als solche durchscheinend und werden erst durch Pigmente optisch opak gemacht bzw. eingefärbt. Aufgrund der hohen Belastung durch Schweiß und allgemein Körperflüssigkeiten werden die orthopädisch-medizinisch verwendeten Kunststoffe schnell unansehnlich, was insbesondere für die stark feuchtigkeitspermeablen Kunststoffe und Gelpolymere gilt. Gegen die bei pigmentierten oder gefärbten Kunststoffen unvermeidlichen Verfärbungen helfen bislang nur starke Pigmentierungen und Einfärbungen im geeigneten Lichtabsorptionsbereich. Aus materialtechnischen Gründen sind hohe Mengen an Farb- und Pigmentzuschlägen jedoch häufig unerwünscht, da sie physikalische und mechanische Eigen-schaften, die für die gewünschte Wirkung des Produktes unerlässlich sind, deutlich verschlechtern können. Ist solchen Linern Silber beigefügt, im Allgemeinen feindispers durch Einmischen in die Kunststoffmasse, werden diese trotz Pigmentierung aufgrund der hohen Belastung durch Schweiß schnell unansehnlich. Hiergegen hilft nur eine Einfärbung in Grau oder Anthrazit bis Schwarz, was häufig nicht erwünscht ist.

Der Erfindung liegt die Aufgabe zugrunde, Verfärbungen an eingefärbten oder pigmentierten Kunststoffteilen zu vermeiden oder besser zu kaschieren und insbesondere Materialien, die für die Verwendung im direkten Hautkontakt mit einem Benutzer vorgesehen sind und als antibakterielles Mittel einen Anteil an fein in dem Material verteiltem Silber enthalten, so auszurüsten, dass das Produkt über lange Gebrauchsdauer optisch praktisch unverändert und ansehnlich bleibt und die Materialeigenschaften dabei nicht beeinträchtigt werden.

Diese Aufgabe wird durch ein orthopädisches Polster gemäß Anspruch 1 gelöst, sowie durch einen orthopädischen Artikel gemäß Anspruch 6.

Allgemein beschreibt die Erfindung die Verwendung von fein verteilten Metallpartikeln, insbesondere der Gruppe Aluminium oder einer Aluminiumlegierung, Magnesium, Bronze, Titan und/oder Platin, in einem Material, vorzugsweise einem Kunststoffmaterial, in dem Silber als antibakterielles Mittel enthalten sein kann, zur Kaschierung von Verfärbungen bzw. der Silberverfärbung bei Gebrauch des Materials an der Luft oder auf der Haut.

Erfindungsgemäß ist die Verwendung in einem orthopädischen Polster.

Unter einem "orthopädischen Polster" soll hier jedes in der Orthopädietechnik verwendete Polster verstanden werden, was für medizinische Zwecke verwendete Polster mit einschließen soll. Bei derartigen Polstern handelt es sich in erster Linie um unterschiedlich geformte Polsterpads, die beispielsweise auch auf die Haut geklebt werden können, um an bestimmten Punkten des Körpers eine Polsterwirkung zu erreichen, um Prothesenauskleidungen, Orthesenpolsterungen, Prothesenschäften, Prothesenliner, Schuheinlegesohlen, orthopädische Schäfte und Schuhe allgemein und orthopädische Kunststoffstrümpfe.

Das Polster kann auch wenigstens einseitig mit einer textilen Auflage versehen sein oder ein- oder beidseitig beschichtet sein.

Besonders bevorzugt ist die Verwendung des erfindungsgemäßen Materials für einen Amputationsstumpfliner oder Gliedmaßen-(Amputations)stumpfliner bzw. ein orthopädisches Polster in Form eines Amputationsstumpfliners. Ein Amputationsstumpfliner bewerkstelligt den polsternden Übergang zwischen einem Gliedmaßenstrumpf und einer Prothesenhalterung, insbesondere einem Protheseschaft, und besteht im Wesentlichen aus einem polymeren, elastischen Material, das hier mit den zusätzlichen Metallpartikeln und, falls gewünscht, zusätzlich mit Silber versetzt ist. An dem Liner können weiterhin Anschlüsse zur Prothese, Verankerungen, Ventile und dergleichen vorgesehen sein. Dies ist dem auf dem Gebiet tätigen Fachmann bekannt und braucht hier nicht näher beschrieben zu werden. Weiterhin kann der Liner einseitig mit einer textilen Auflage versehen oder beidseitig (d.h. entweder nur auf der hautzugewandten Seite, nur auf hautabgewandten Seite oder auf beiden Seiten) beschichtet sein. Bei der Beschichtung kann es sich vorzugsweise um eine dünne Bedampfung handeln, beispielsweise eine CVD-Beschichtung. Bei dem Material des Stumpfliners handelt es sich vorzugsweise um ein homopolymeres oder copolymeres Material oder um eine Gemisch mehrerer Kunststoffe. Der Kunststoff des Liners ist vorzugsweise elastisch, weiter vorzugsweise gelförmig und je nach Anwendungszwecken mit besonderen mechanischen Eigenschaften ausgestattet.

Neben den Stumpflinern sind folgende orthopädische Polster besonders geeignet, um erfindungsgemäß ausgerüstet zu werden: allgemein orthopädische Polsterpads, Prothesenauskleidungen, Orthesenpolsterungen, Prothesengeschäfte, Teile von Stützapparaten, Schuheinlegsohlen oder orthopädische Kunststoffstrümpfe, wie sie als Gewirke ähnlich wie Liner zur Polsterung orthopädischer Halterapparate oder Prothesen eingesetzt werden.

Allgemein umfasst die Erfindung auch die Verwendung der orthopädischen erfindungsgemäßen Polster in komplexeren orthopädischen Artikeln bzw. für orthopädische Artikel, sowie orthopädische Artikel, die mit dem erfindungsgemäßen Polster ausgestattet sind.

Bei den Metallen, die in Ergänzung zu Silber oder ohne Silber verwendet werden, kann es sich um Metalle in nanoskaliger oder kolloidaler Form, um Pulverteilchen, feines Granulat, Blättchen oder Schuppen, Späne oder Fäden handeln. Gängige kommerziell angebotene Metallpulver sind für die Erfindung geeignet. Entsprechende Pulver werden beispielsweise auch in der Schmuck- oder Farbenindustrie verwendet. Die Teilchengrößen liegen im Allgemeinen zwischen 1 und 100 µm, bei blattförmigen und nanoskaligen Teilchen liegt wenigstens die Teilchendicke im Bereich ab 0,1 µm.

Die erfindungsgemäß verwendeten Metallteilchen besitzen keine schwarzen Oxide oder -sulfide, der durch sie erzeugte metallische Farbeffekt ändert sich während der Benutzung der aus dem Material hergestellten Produkte praktisch nicht. Überraschenderweise wurde festgestellt, dass im Vergleich zu Pigmenten als allgemeine Mittel gegen Vergilbung wesentlich geringere Mengen, nämlich nur zwischen 5 und 10 % des für einen opaken Effekt wenigstens erforderlichen Pigmentgehalts oder Farbstoffgehalts notwendig sind, um einen zur Kaschierung der Verfärbung bei Kontakt mit der Haut eines Benutzers und mit Schweiß insbesondere der Silberverfärbung ausreichenden Effekt zu erzielen. Bevorzugt werden Teilchen aus Aluminium oder einer Aluminiumlegierung verwendet. Der Einfärbung des Kunststoffs wird durch das gleichzeitige Vorhandensein von Metallteilchen und Farb- oder Pigmentteilchen eine größere Strahlkraft verliehen. Sehr ansprechende optische Effekte ergeben sich auch bei der Verwendung transluzenter Farben in Kombination mit den erfindungsgemäßen Metallpartikeln.

Vorzugsweise sollte der Metallgehalt, d.h. der Anteil an Metallpartikeln auf das Gesamtgewicht bezogen, bei Anwesenheit von Silber in Ergänzung des Silbergehalts, wenigstens 0,01 Gew.-%, weiter vorzugsweise wenigstens 0,1 Gew.-% und insbesondere wenigstens 0,5 Gew.-% betragen. Ein Wert bis 1 Gew.-% oder maximal 3 Gew.-% wird als ausreichend angesehen, um den gewünschten Effekt zu erzielen. Der in antibakteriell mit Silber ausgerüsteten Materialen (Kunststoffen und Textilien) eingesetzte Silbergehalt beträgt vorzugsweise unter 0,01 Gew.-% bis maximal 0,1 Gew.-%.

Ein wesentlicher Vorteil der Erfindung liegt bei der Anwendung in Kunststoffen daher bereits darin, dass weniger Additiv für die Einfärbung verbraucht wird, wenn Pigmente/Farben und Metall verwendet werden, als wenn nur Pigmente oder Farben eingesetzt würden. Gegebenenfalls kann auf die Verwendung von Pigmenten und Farben auch ganz verzichtet werden.

Ein weiterer noch bedeutsamer Vorteil wird darin gesehen, dass der Metallzusatz in solch geringer Menge praktisch keinen Einfluss auf die mechanischen Eigenschaften eines Kunststoffteils besitzt, was insbesondere für gelartige und weiche Kunststoffe von Bedeutung ist. Dies ist bei funktionellen Produkten, bei denen es auf die Werkstoffeigenschaften entscheidend ankommt, von besonders großer Bedeutung. Gerade Kunststoffpolster, die in der Medizin und für die Orthopädietechnik gebraucht werden, Orthesenteile, Teile von Stützapparaten und ähnliche Produkte sollen ganz bestimmte elastische Eigenschaften, Festigkeiten, Dehnbarkeiten usw. besitzen - gegebenenfalls auch anisotrop - und diese sollen durch Zuschlagsstoffe nicht verschlechtert werden.

Ein zusätzlicher Vorteil der Erfindung ist darin zu sehen, dass das Kunststoffmaterial eine silberne oder bronzene Einfärbung erhält und damit einen optischen Charakter, der äußerst ansprechend ist. Dies ist besonders ausgeprägt bei gleichzeitiger Anwesenheit von Farbpigmenten außer weiß und schwarz und bei transluzenten Pigmenten.

Es wurde außerdem beobachtet, dass die Metallisierung, ggf. zusätzlich zu Silber, beim Benutzer einen positiven Effekt im Hinblick auf Nervenschmerzen und Phantomschmerzen hervorrufen kann. Dies könnte auf die kapazitive Wirkung des eingesetzten Metalls zurückzuführen sein.

Da die Erfindung bei Produkten angewendet wird, die gleichzeitig Silber enthalten, ist es überraschend, dass auch bei feuchtigkeitsdurchlässigen und quellfähigen Kunststoffen und Textilien offenbar keine Lokalelemente gebildet werden. An sich wäre zu vermuten, dass die in der Spannungsreihe unter Silber stehenden Elemente wie z. B. Aluminium gebildete Silberionen, die eine antibakterielle Wirkung entfalten sollen, reduzieren und so diese Wirkung zunichte machen könnten. In der Spannungsreihe über Silber stehende Elemente könnten hingegen das Silber zu schnell oxidieren und verbrauchen. Beides geschieht, wie experimentell festgestellt werden konnte, nicht. Die Silberkonzentration und die Silberwirkung bleiben erhalten, selbst wenn Quellversuche mit den silberdotierten Materialien durchgeführt werden. So konnten an silberdotierten Silikonlinern, die eine Woche in Salzlösung gelegen hatten, keine Silberverluste nachgewiesen und keine Leitfähigkeiten gemessen werden. Es ist daher nicht zu befürchten, dass das erfindungsgemäße Material seine Wirkung bei Kontakt mit Hautfeuchtigkeit oder Schweiß verliert. Den oben genannten Vorteilen stehen demnach keine Nachteile durch Fremdmetalle gegenüber.

Als Basismaterial, welches ggf. mit Silber und dem zusätzlichen Metall, das in erster Linie als Metallfarbe wirken soll, versetzt wird, sind u.a. Polymere (natürliche oder künstliche, d.h. Kunststoffe aller Art, Gummi, Latex, Guttapercha) und Textilien (Naturfaserstoffe und - vliese, Kunstfaserstoffe und -vliese) geeignet, aber auch andere Materialien, die selbst keine Metalle sind und vorzugsweise nicht bzw. nicht in wesentlichem Maße elektrisch leiten, z.B. Keramiken.

Für die Verwendung besonders geeignete Kunststoffe sind polymere oder copolymere elastische Kunststoffe, Weichkunststoffe oder Polymergele.

Besonders bevorzugt ist ein erfindungsgemäßes Material, das für die Verwendung im direkten Hautkontakt eines Benutzers vorgesehen ist und einen Anteil an fein in dem Material verteiltem Silber als antibakterielles Mittel enthält, wobei es neben dem Silberanteil einen Anteil an fein in dem Material verteilten Metallen der Gruppe Aluminium, Magnesium, Zink, Bronze, Titan und Platin, einzeln oder in Kombination, vorzugsweise aus Aluminium oder einer Aluminiumlegierung, enthält.

Die Erfindung umfasst allgemein auch eine Hautauflage aus dem erfindungsgemäßen Material. Hierbei kann es sich um jede Hautauflage handeln, die auch im Stand der Technik bereits mit Silber ausgerüstet wurde, insbesondere um Polsterpads, Prothesenauskleidungen, Orthesenteile, Teile von Stützapparaten, textile Auflagen, wie elastische Binden, Amputationsstumpfliner, Prothesenschäfte, Prothesenhalterungen und Hautauflagen zur Wärmeisolation. Besonders bevorzugt ist die Verwendung des erfindungsgemäßen Materials für einen Amputationsstumpfliner bzw. eine Hautauflage in Form eines Amputationsstumpfliners.

Ferner umfasst die Erfindung einen orthopädischen Artikel mit dem Material als ein Polster oder Wärmeschutzmaterial insbesondere für orthopädische Zwecke und umfasst weiterhin die Verwendung des Materials für Halterungen und Stützapparate am menschlichen Körper, Orthesen und Orthesenteile, Schaftgießharze, Textilbeschichtungen und zum Tränken von Textilien.

Weitere Produkte, die vorteilhaft aus oder mit dem erfindungsgemäßen Material hergestellt werden können, sind Einlegsohlen, Sitzpolster für Stühle, Rollstühle und Betten, Armauflagen, Stützstrümpfe und Socken.

Im Folgenden wird die Erfindung anhand von Beispielen näher beschrieben, der der Illustration dienen sollen.

Die nachfolgend beschriebenen Polymerrezepturen wurden als Materialgrundlage verwendet. Zu der Materialgrundlage oder dem Basismaterial wurde Silber in der gewünschten Form zugefügt und/oder zusätzlich wenigstens ein Metall der Gruppe Aluminium, Magnesium, Zink, Titan, Platin oder Bronze. Der Silberzusatz erfolgte bei allen Materialien / Proben mit Hilfe eines kommerziell erhältlichen Additives mit 1,6 Gew.-% Silberanteil, das in einer Menge von 0,6 Gew.-% bezogen auf die Materialgrundlagen zugesetzt wurde.
I. Als Materialgrundlagen sind beispielsweise geeignet und wurden verwendet:
   1.) Silikone:
      RTV-Silikongele (Zwei-Komponenten-Gele Polyaddition), z.B. Rhodia^{®} 4411 oder 4420, gegebenenfalls versetzt mit 1 bis 15 Gew.-% Silikonfluid oder Silikonöl
      Geeignete Rezepturen für Silikonmaterialien sind insbesondere:
         Rhodia^{®} 4411 100:12
         Rhodia^{®} 4420 100:10
         Rhodia^{®} 4420 100:10 mit 5 % Silikonfluid 0,65 cst
         Dow Corning Silastic^{®} T2 100:10, ohne oder mit 10 % Dow Silikonöl 200 (350 cst)
         Wacker Elastosil^{®} RTV 4644 100:10 mit oder ohne 15 % Silikonöl Wacker AK 1000
         jeweils vermischbar mit optimal 0,05 bis 0,15 Gew.-% Al-Pulver
   2.) Polyurethane:
      Polyurethan aus Polyetherpolyol mit aliphatischem Isocyant 100:13; 0,1 bis 0,5 % Coscat^{®}-Katalysator, Viskositätsverminderer BYK A 535; oder Conathan TU 401 mit TU 810 100:51
      jeweils vermischbar mit optimal 0,05 bis 0,15 Gew.-% Al-Pulver
   3.) Block-Copolymere:
      SEBS:
         15 - 30 % Kraton 1651 G, 60 bis 75 % med. Weißöl, 0 - 1 % Antioxidant, 0 - 25 % Viskositätsverminderer (z.B. Waschbenzin)
      SEEPS:
         10 - 20 % Septon 4044, 70 - 80 % med. Weißöl, 0 - 1 % Antioxidant, 0 - 20 % Viskositätsverminderer (z.B. Waschbenzin)
         jeweils vermischbar mit optimal 0,01 bis 0,2 Gew.-% Al-Pulver.
II. An folgenden Rezepturen wurden Vergleichsmessungen zur Beeinflussung der mechanischen Eigenschaften durch Pigmente und Metallpartikel vorgenommen. Die Ergebnisse sind in Tabelle 1 dargestellt.

(Materialgrundlage mit Siberzusatz, wie oben angegeben)
Rezeptur A: Rhodia 4411 100:12 (Referenz, ohne Metall, ohne Pigment)
Rezeptur B: Rhodia 4411 100:12 mit 1 Gew.-% Elastosil Farbpigment FL
Rezeptur C: Rhodia 4411 100:12 mit 3 Gew.-% Elastosil Farbpigment FL
Rezeptur D: Rhodia 4411 100:12 mit 0,1 Gew.-% Al-Pulver

| | Rezeptur A (Referenz) | Rezeptur B (1 % Pigment) | Rezeptur C (3 % Pigment) | Rezeptur D (0,1 % Al-Pulver |
|---|---|---|---|---|
| Zugfestigkeit (N/mm²) | 1,36 | 1,45 | 1,20 | 1,41 |
| Bruchdehnung (%) | 522 | 539 | 480 | 585 |
| 400 % Spannung (N/mm²) | 0,8572 | 0,8578 | 0,9017 | 0,7184 |
| Weiterreißwiderstand (N/mm) | 7,12 | 7,46 | 6,90 | 7,86 |
| Koeffizient | 1,59 | 1,69 | 1,33 | 1,96 |

### Diskussion der Ergebnisse der Vergleichsmessungen:

Rezeptur B enthielt 1 Gew.-% Farbpigment, was als niedrigste erforderliche Konzentration zur Abdeckung einer (leichten) Vergrauung durch Silber anzusehen ist. Bei stärkeren Verfärbungen, wie sie durch Kontakt mit Schwefelverbindungen möglich sind, ist im Silber basierten Liner eine deutlich höhere Farbkonzentration von mindestens 3 % erforderlich (siehe Rezeptur C). In beiden Fällen, Rezepturen B und C, wurde das gleiche graue Farbpigment verwendet. Der visuell festgestellte Deckungsgrad entsprach beim Rezepturbeispiel D mit 0,1 Gew.-% Aluminiumpulver etwa dem bei Rezeptur C mit 3 Gew.-% Farbpigment.

Aus den Daten ist zu erkennen, dass das Metallpulver die mechanischen Eigenschaften nicht zu sehr beeinträchtigt. Der Einsatz von Metallpulver kann daher in optischer Hinsicht wie auch bezüglich der Materialeigenschaften als sehr vorteilhaft angesehen werden.

Alle Rezepturen können mit kommerziell erhältlichen Metallpulvern wie für die Druck- und Lackfarbenindustrie oder die Galvanotechnik im Gebrauch angesetzt werden.

Alle Farbeffekte und mechanischen Eigenschaften werden entsprechend für die gleichen Rezepturen, nur ohne Silberanteil gefunden. Der visuell festgestellte Deckungsgrad führt zu einer sicheren Abdeckung von Kunststoffverfärbungen durch Hautkontakt und insbesondere durch Schweiß.

## Patentansprüche

1. Orthopädisches Polster, insbesondere ein Amputationsstumpfliner, ein Polsterpad, eine Prothesenauskleidung, ein Orthesenpolster, ein Prothesenschaft, eine Schuheinlegesohle oder ein orthopädischer Strumpf,
mit einem Kunststoffmaterial, in dem Silber als antibakterielles Mittel enthalten ist und in dem Metallpartikel, insbesondere der Gruppe Aluminium oder einer Aluminiumlegierung, Magnesium, Bronze, Titan und/oder Platin fein verteilt sind, um Verfärbungen bei Gebrauch des Materials auf der Haut zu kaschieren.

2. Orthopädisches Polster nach Anspruch 1, wobei das Polster wenigstens einseitig mit einer textilen Auflage versehen ist oder ein- oder beidseitig beschichtet ist.

3. Orthopädisches Polster nach Anspruch 1 oder 2, **dadurch gekennzeichnet, dass** die Metallpartikel zur Kaschierung der Verfärbung als Pulverteilchen, feine Späne oder Fäden vorliegen.

4. Orthopädisches Polster nach einem der Ansprüche 1 bis 3, **dadurch gekennzeichnet, dass** das Kunststoffmaterial aus einem polymeren oder copolymeren elastischen Kunststoff, Weichkunststoff oder Polymergel besteht.

5. Orthopädisches Polster nach einem der Ansprüche 1 bis 4, **dadurch gekennzeichnet, dass** der Metallgehalt in Ergänzung zu Silber wenigstens 0,01 Gew.-% vorzugsweise wenigstens 0,1 Gew.-%, weiter vorzugsweise wenigstens 0,5 Gew.-% beträgt.

6. Orthopädischer Artikel, ausgestattet mit einem orthopädischen Polster nach einem der vorstehenden Ansprüche.

## Claims

1. Orthopedic cushion, specifically an amputation stump liner, a cushioning pad, a prosthesis covering, an orthotic cushion, a prosthesis shaft, a shoe sole insert, or an orthopedic stocking, having a plastic material which contains silver as an antibacterial agent and in which metal particles, specifically of the group aluminum or , an aluminum alloy, magnesium, bronze, titanium, and/or platinum, are finely dispersed in order to conceal discoloration on use of the material on the skin.

2. Orthopedic cushion according to Claim 1, in which the cushion is coated on at least one side with a textile cover or is coated on one or both sides.

3. Orthopedic cushion according to Claim 1 or 2, **characterized in that** the metal particles for concealing the discoloration are in the form of powder particles, fine chips, or filaments.

4. Orthopedic cushion according to one of Claims 1 to 3, **characterized in that** the plastic material is composed of a polymeric or copolymeric elastic plastic, soft plastic, or polymer gel.

5. Orthopedic cushion according to one of Claims 1 to 4, **characterized in that** the metal content as a supplement to silver is at least 0.01% by weight, and preferably at least 0.1% by weight, with a weight of at least 0.5% by weight being even more preferable.

6. Orthopedic article, equipped with an orthopedic cushion according to one of the preceding claims.

## Revendications

1. Matelassage orthopédique, en particulier doublure de moignon d'amputation, plage de matelassage, habillage de prothèse, matelassage d'orthèse, tige de prothèse, semelle interne de chaussure ou bas orthopédique,
comprenant un matériau synthétique dans lequel de l'argent est contenu en tant qu'agent antibactérien et dans lequel des particules métalliques, en particulier du groupe de l'aluminium ou d'un alliage d'aluminium, du magnésium, du bronze, du titane et/ou du platine sont finement réparties, afin de cacher des décolorations lors de l'utilisation du matériau sur la peau.

2. Matelassage orthopédique selon la revendication 1, dans lequel le matelassage est pourvu au moins d'un côté d'un support textile ou est revêtu d'un côté ou des deux côtés.

3. Matelassage orthopédique selon la revendication 1 ou 2, **caractérisé en ce que** les particules métalliques destinées à cacher la décoloration se présentent sous forme de particules de poudre, de fins copeaux ou de fils.

4. Matelassage orthopédique selon l'une quelconque des revendications 1 à 3, **caractérisé en ce que** le matériau synthétique se compose d'une matière synthétique élastique polymère ou copolymère, d'une matière synthétique souple ou d'un gel polymère.

5. Matelassage orthopédique selon l'une quelconque des revendications 1 à 4, **caractérisé en ce que** la teneur en métal en complément de l'argent est d'au moins 0,01 % en poids, de préférence d'au moins 0,1 % en poids, plus préférablement d'au moins 0,5 % en poids.

6. Article orthopédique comprenant un matelassage orthopédique selon l'une quelconque des revendications précédentes.
